# EUROPEAN PATENT APPLICATION

(11) **EP 3 398 623 A1**
(43) Date of publication of application: **07.11.2018**
(21) Application number: 16880442.5
(22) Date of filing: 17.06.2016
(51) Int. Cl.: A61L 31/16, A61L 31/02, A61L 31/10

(54) **IMPLANTABLE MEDICAL INSTRUMENT MADE OF IRON-BASED ALLOY AND MANUFACTURING METHOD THEREFOR**

(30) Priority: 29.12.2015 CN 201511024701
(71) Applicant: Lifetech Scientific (Shenzhen) Co., Ltd., Shenzhen, Guangdong 518057 (CN)
(72) Inventor: SUN, Hongtao, Shenzhen Guangdong 518057 (CN); CHEN, Liping, Shenzhen Guangdong 518057 (CN); HU, Jun, Shenzhen Guangdong 518057 (CN); ZHANG, Deyuan, Shenzhen Guangdong 518057 (CN)
(74) Representative: Prinz & Partner mbB
(86) International application number: PCT/CN2016/086118
(87) International publication number: WO 2017/113634

(57) **Abstract**

An implantable medical instrument is made of an iron-based alloy, comprising an iron-based alloy substrate and a drug-loaded coating. The drug-loaded coating comprises a polymer and an active drug; the weight average molecular weight of the polymer is greater than or equal to 50,000 and less than or equal to 1,000,000; micro-pores having a diameter of less than or equal to 10 micrometers are formed in the drug-loaded coating; the percentage of the active drug released on the implantable medical instrument made of an iron-based alloy is greater than or equal to 4t^{1/2}-1 and less than or equal to 6.9t^{1/2}+63, wherein t ∈ (0, 28], and t represents sampling time/days.

## Description

### Technical Field

The present application belongs to the field of degradable implantable medical devices, and relates to a controlled drug release iron-based alloy drug-loaded implantable medical device and a manufacturing method therefor.

### Background Art

At present, implantable medical devices are generally made of a metal and its alloy, ceramic, a polymer and relevant composite materials, wherein a metal material-based implantable medical device is particularly popular due to its excellent mechanical properties such as high density and high toughness.

As a crucial element in the human body, iron participates in many biochemical processes, such as the delivery of oxygen. Corrosion-prone pure iron stents which are made by Peuster M by adopting a laser engraving method and having shapes similar to those of clinically-used metal stents were implanted into descending aortas of 16 New Zealand rabbits. This animal experimental result has shown that no thrombotic complications occurred within 6 to 18 months, and no adverse events occurred either. Pathological examination has confirmed that a local blood vessel wall did not have inflammatory reaction and no obvious proliferation of smooth muscle cells occurred. It is preliminarily demonstrated that the degradable iron stents are safe and reliable, and have good prospects for use.

In terms of clinical use, when the absorbable implantable medical device fulfills its expected use, after a diseased portion is cured and has recovered to its normal shape and function (cured), so as to not cause a new biological compatibility problem, it is desirable to minimize the time needed the device to completely corrode or degrade and to be absorbed by the human vessel or organ. According to different clinical applications for the device, the recovery period is generally considered to be 1 to 6 months, and within this period of time, the device is required to provide structural integrity and to have a sufficient mechanical property. An iron-based alloy has a good biological compatibility, but due to the slow corrosion of the iron-based alloy in the body, an iron-based alloy device can take a long time to be completely corroded after the recovery period; as a result, it is necessary to accelerate the corrosion of the iron-based alloy to shorten the corrosion cycle of the iron-based alloy device. It has been reported that the corrosion of the iron-based alloy may be accelerated by coating the surface of the iron-based alloy (including pure iron and a medical iron-based alloy) with a degradable polyester coating.

A degradable polymer drug-loaded coating is disposed on the implantable medical device, so that it may further exert a drug therapy effect after the device is implanted into the body. For example, if an active drug component for treating restenosis is added on a bare stent, the lumen restenosis rate may be greatly reduced after the stent is implanted into a blood vessel. The release speed of an active drug is crucial for providing a therapeutic effect of the drug-loaded implanted medical device. When the total drug amount of the device is in a certain range, if the drug is released too slowly, its therapeutic effect is limited, but if the drug is released too quickly, extremely high local drug concentration could cause toxic and side effects. Therefore, the iron-based alloy implantable medical device must have a proper drug release speed in addition to a proper corrosion speed, as set forth in the patent application CN201310533266.6 filed by this applicant on October 31, 2013.

### Summary of the Invention

For the purpose of solving the technical problems, in view of the shortcomings in the prior art, the present application provides an iron-based alloy implanted medical device. By use of a polymer within a specific molecular weight range as a drug carrier of a drug-loaded coating, controlled drug release can be achieved after the iron-based alloy implantable medical device is implanted in the body.

The iron-based alloy implantable medical device includes an iron-based alloy substrate and a drug-loaded coating. The drug-loaded coating includes a polymer and an active drug. The weight average molecular weight of the polymer is more than or equal to 50,000 and less than or equal to 1,000,000, and micro-pores having an aperture not more than 10 microns are formed in the drug-loaded coating. The weight average molecular weight of the polymer is preferably more than or equal to 100,000 and less than or equal to 500,000, and the aperture of the micro-pores is not more than 1 micron, and further not more than 0.1 micron. The amount of the active drug on a unit area of the iron-based alloy substrate is more than or equal to 5 µg/cm² and less than or equal to 500 µg/cm², and further more than or equal to 50 µg/cm² and less than or equal to 300 µg/cm². A mass ratio of the polymer to the active drug is more than or equal to 50: 1 and less than or equal to 0.1:1, and further more than or equal to 10:1 and less than or equal to 0.2:1

The thickness of the drug-loaded coating is more than or equal to 2 microns and less than or equal to 50 microns. Further, when the micro-pores having the aperture not more than 1 micron are formed in the drug-loaded coating, the thickness of the drug-loaded coating is more than or equal to 5 microns and less than or equal to 30 microns. The drug-loaded coating may be one layer or a multilayer combination, or may be a uniform coating or a non-uniform coating such as an asymmetric coating, a non-continuous coating or a single-surface coating. At least part of the surface of the iron-based alloy substrate may be coated with the drug-loaded coating. When an iron-based alloy has a gap, a groove or a cavity, besides the surface of the iron-based alloy substrate, the gap, the groove or the cavity may be also coated with the drug-loaded coating.

The polymer is a degradable polyester polymer, or a mixture of the degradable polyester polymer and a non-degradable polyester polymer, or a copolymer of at least one monomer forming the degradable polyester polymer and at least one monomer forming the non-degradable polyester polymer. The degradable polyester polymer is selected from the group consisting of polylactic acid, polyglycolic acid, poly(ethylene succinate), poly(P-hydroxybutyrate), polycaprolactone, polyethylene glycol adipate, poly-pentanoate, poly-hydroxyl alkyl alcohol ester and poly-malate, or a copolymer of at least two of monomers forming the degradable polyester polymer; the non-degradable polyester polymer is selected from the group consisting of starch, chitosan, cellulose, glycan, glycan and a derivative thereof, polyurethane (PU), polycarbonate, polymethyl methacrylate (PMMA), polystyrene (PS), polybutylene, poly-butyl-methacrylate (PBMA) and polyacrylamide, or a copolymer of at least two of monomers forming the non-degradable polyester polymer.

The active drug is selected from the group consisting of a vascular proliferation inhibition drug, an antiplatelet drug, an antithrombotic drug, an anti-inflammation reaction drug and an antiallergic drug. The vascular proliferation inhibition drug is selected from the group consisting of taxol, sirolimus and a derivative thereof; the antiplatelet drug is cilostazol; the antithrombotic drug is heparin; the anti-inflammation reaction drug is dexamethasone; and the antiallergic drug is selected from the group consisting of diphenhydramine, chlorpheniramine, promethazine, hydrocortisone, triamcinolone, methylprednisolone, clarityne, fexofenadine, levocetirizine, mizolastine and ebastine.

The iron-based alloy implantable medical device may be a blood vessel stent, a non-vascular endovascular stent, an occluder, and other cardiovascular implants, orthopaedic implants, gynecological implants, andrological implants and respiratory implants.

The iron-based alloy substrate is an iron-based alloy with the carbon content not more than 2.11 wt.% or pure iron.

The release percentage of the active drug is more than or equal to 4t^{1/2}-1 and less than or equal to 6.9t^{1/2}+63, and t is more than 0 and less than or equal to 28, and represents sampling time/days.

The present application further provides a manufacturing method of an iron-based alloy implantable medical device, including: dissolving a polymer and an active drug in an organic solvent to form a solution, and then coating an iron-based alloy substrate with the solution, for example, coating at least part of the surface, a gap, a groove or a cavity with the solution, wherein the weight average molecular weight of the polymer is more than or equal to 50,000 and less than or equal to 1,000,000, and the organic solvent is selected from the group consisting of trichloromethane, dichloromethane, ethyl acetate, tetrahydrofuran, acetone, methanol, ethanol, acetonitrile, 1,4-dioxane, dimethyl formamide and isopropanol.

Compared with the prior art, the iron-based implantable medical device of the present application has a proper drug release speed due to the selection of the polymer with the molecular weight more than or equal to 50,000 and less than or equal to 1,000,000 as a drug carrier, and due to the fact that micro-pores having an aperture not more than 10 microns are formed in the drug-loaded coating, the release percentage of the active drug is more than or equal to 4t^{1/2}-1 and less than or equal to 6.9t^{1/2}+63, wherein t is more than 0 and less than or equal to 28, and represents sampling time/days.

### Detailed Description of the Invention

For the purpose of facilitating understanding of the present application, the present application provides preferred embodiments, but the present application may be implemented in many different forms, and is not limited by the embodiments described herein. On the contrary, the objective of providing these embodiments is to disclose the contents of the present application more thoroughly and comprehensively.

Unless otherwise specified, all technical and scientific terms used herein are the same as meanings of general understandings of persons skilled in the art of the present application. The terms used in the description herein are merely to describe the specific embodiments, but are not intended to limit scope of the present application.

The release speed of an active drug in a drug-loaded coating of an iron-based alloy implantable medical device of the present application is affected by various factors, including:
(1) the higher the molecular weight of a polymer, the more obvious the phase separation between the polymer and the drug, and the faster the drug release;
(2) the smaller the ratio of the polymer to the drug, the lower the restriction capacity of the polymer to the drug, and the faster the drug release;
(3) the thinner the drug-loaded coating, the shorter a drug diffusion release path, and the faster the drug release;
(4) the higher the saturated vapor pressure of a solvent used in a manufacturing method, the faster its volatilization, and the weaker the mutual action force between the polymer and the drug, and the faster the drug release.

From a drug release mechanism, the drug release is mainly accomplished by techniques such as dissolving, diffusion, degradation of the drug carrier (such as the polymer) and the like. The size of the aperture in the drug-loaded coating directly affects the dissolving and diffusion speeds of the drug, and also affects the degradation speed of the polymer. Therefore, the present application controls the drug release speed by directly controlling the molecular weight range of the polymer and the aperture of the drug-loaded coating. The present application may obtain drug-loaded coatings with different aperture sizes by selecting the polymer with a weight average molecular weight of more than or equal to 50,000 and less than or equal to 1,000,000, and adjusting the molecular weight of the polymer, the types of the drug and a solvent, and a ratio of the polymer to the drug within this range. The size of the aperture of the drug-loaded coating is irrelevant to a substrate.

The drug release speed of the device of the present application is represented by an animal experiment. An iron-based alloy device with the loaded drug mass of S is implanted into an abdominal aorta of a rabbit; and then the device and a tissue where the device is implanted are cut out at a preset time, and the residual drug in the device and the tissue is subjected to constant volume and ultrasonic treatment with an extracting solvent (such as acetonitrile) with the volume of V to enable the drug to be completely dissolved in the extracting solvent, thus obtaining a drug extracting solution. A drug concentration c of the drug extracting solution is measured by an Agilent 1260 high performance liquid chromatography, thereby obtaining the residual drug mass S1=cV of a sample, and then the drug release percentage of the sample at this time point is Y%=(S-S1)/S*100%.

A further detailed description is made to the present application in combination with the specific embodiments below by taking a drug-loaded iron-based alloy stent as an example, but this is not intended to limit the scope of protection of the present application.

In all the embodiments below, the drug-loaded stent is implanted into the abdominal aorta of the rabbit, and then samples are respectively taken on the 1st day, the 7th day, the 14th day and the 28th day. If the drug release percentages Y% of the samples are respectively within the ranges of 3 to 70 percent, 9 to 81 percent, 14 to 89 percent and 20 to 99 percent, it is considered that the stent has a controllable drug release property.

The molecular weight of a high-molecular weight polymer is the weight average molecular weight, and its size is detected with an eight-angle laser light scattering instrument produced by the Wyatt Technology Corporation.

A method for testing the aperture of the drug-loaded coating mainly includes: obtaining an intact coating section of the original drug-loaded coating stent, then observing a pore in the coating with a scanning electron microscope and measuring the diameter of the pore. However, If the apparent aperture cannot be observed by scanning electron microscope magnification of 8000 times, it is deemed that the aperture is less than 0.1 micron.

### Embodiment 1

Ethyl acetate is used as a solvent; polylactic acid-glycollic acid with the weight average molecular weight of 100,000 and taxol are mixed at a mass ratio of 3:1 and dissolved in the ethyl acetate to form a solution; the surface of a pure iron stent substrate is coated with the solution, thereby forming a drug-loaded coating with a thickness of 15 µm and an aperture size of 0.2 µm; and the amount of the taxol on the area of the stent substrate is 200 µg/cm². The manufactured drug-loaded stents are implanted into the abdominal aorta of rabbits respectively, and samples are respectively taken on the 1st day, the 7th day, the 14th day and the 28th day and then are tested, the drug release percentages of the stent are 20 percent, 40 percent, 50 percent and 65 percent respectively. The experimental result shows that the drug-loaded coating of the stent manufactured in this embodiment has the controllable drug release property.

### Embodiment 2

Poly(ethylene succinate) with a weight average molecular weight of 200,000 and sirolimus are respectively dissolved in a trichloromethane solution at a ratio of 4:1; the surface of a stent is sprayed with the solution, thus a poly(ethylene succinate)-sirolimus drug-loaded coating with a thickness of about 5µm and an aperture of less than 0.1µm is formed; and the amount of the sirolimus on the area of a stent substrate is 50 µg/cm²; and the surface of the drug-loaded coating of an iron-zinc alloy stent substrate is coated with a trichloromethane solution of poly(ethylene succinate) with a molecular weight of 60,000 to form a top layer with a thickness of 10 µm and an aperture of less than 0.1 µm. The manufactured drug-loaded stents are implanted into the abdominal aorta of rabbits respectively, and samples are respectively taken on the 1st day, the 7th day, the 14th day and the 28th day and then are tested, the drug release percentages are 15 percent, 35 percent, 50 percent and 65 percent, respectively. The experimental result shows that the drug-loaded coating of the stent manufactured in this embodiment has the controllable drug release property.

### Embodiment 3

Acetone is used as a solvent; poly-dl-lactic acid with the weight average molecular weight of 300,000, polyglycolic acid and dexamethasone are dissolved in the acetone at a mass ratio of 1:1:1 to form a solution; the inside of a cavity of a nitrided iron-based alloy stent substrate is coated with the solution, thereby forming a drug-loaded coating with a thickness of 25 µm and an aperture of 2 µm; and the amount of the active drug on the area of the stent substrate is 300 µg/cm². The manufactured drug-loaded stents are implanted into the abdominal aorta of rabbits respectively, and samples are respectively taken on the 1st day, the 7th day, the 14th day and the 28th day and then are tested, the drug release percentages are 40 percent, 50 percent, 60 percent and 80 percent, respectively. The experimental result shows that the drug-loaded coating of the stent manufactured in this embodiment has the controllable drug release property.

### Embodiment 4

Tetrahydrofuran is used as a solvent; a polycaprolactone and styrene copolymer with a weight average molecular weight of 1,000,000, taxol and promethazine are dissolved in the tetrahydrofuran at a mass ratio of 10:1:1 to form a solution; the surface of a galvanized iron stent substrate is coated with the solution, thereby forming a drug-loaded coating with a thickness of 50 µm and an aperture of 5 µm; and the amount of the active drug on the area of the stent matrix is 100 µg/cm². The manufactured drug-loaded stents are implanted into the abdominal aorta of rabbits respectively, and samples are respectively taken on the 1st day, the 7th day, the 14th day and the 28th day and then are tested, the drug release percentages are 20 percent, 30 percent, 40 percent and 60 percent, respectively. The experimental result shows that the drug-loaded coating of the stent manufactured in this embodiment has the controllable drug release property.

### Embodiment 5

Ethyl acetate is used as a solvent; poly(ethylene succinate) with a weight average molecular weight of 600,000, levocetirizine and sirolimus are dissolved in the ethyl acetate at a mass ratio of 100:1:1 to form a solution; a gap of a ferro-manganese stent substrate is coated with the solution, thereby forming a drug-loaded coating with a thickness of 10 µm and an aperture of 0.1 µm; and the amount of the active drug on the area of the stent substrate is 10 µg/cm². The manufactured drug-loaded stents are implanted into the abdominal aorta of rabbits respectively, and samples are respectively taken on the 1st day, the 7th day, the 14th day and the 28th day and then are tested, the drug release percentages are 10 percent, 25 percent, 35 percent and 45 percent, respectively. The experimental result shows that the drug-loaded coating of the stent manufactured in this embodiment has the controllable drug release property.

### Embodiment 6

Tetrahydrofuran is used as a solvent; a poly-pentanoate-starch copolymer with a weight average molecular weight of 50,000 and triamcinolone are dissolved in the tetrahydrofuran at a mass ratio of 1:5 to form a solution; only the surfaces of the outer wall and the side wall of an iron-titanium alloy stent substrate are coated with the solution, thus a single-surface drug-loaded coating with a thickness of 15 µm and an aperture of 0.8 µm is formed; and the amount of the active drug on the area of the stent substrate is 200 µg/cm2. The manufactured drug-loaded stents are implanted into the abdominal aorta of rabbits respectively, and samples are respectively taken on the 1st day, the 7th day, the 14th day and the 28th day and then are tested, the drug release percentages are 45 percent, 55 percent, 65 percent and 90 percent, respectively. The experimental result shows that the drug-loaded coating of the stent manufactured in this embodiment has the controllable drug release property.

### Embodiment 7

Dichloromethane is used as a solvent; a polyglycolic acid-cellulose copolymer with a weight average molecular weight of 1,000,000 and sirolimus are dissolved in the dichloromethane at a mass ratio of 1:1 to form a solution; the surface of an iron-cobalt alloy stent substrate is coated with the solution, thereby forming a drug-loaded coating with a thickness of 30 µm and an aperture of 0.5 µm; and the amount of the active drug on the area of the stent matrix is 100 µg/cm2. The manufactured drug-loaded stents are implanted into the abdominal aorta of rabbits respectively, and samples are respectively taken on the 1st day, the 7th day, the 14th day and the 28th day and then are tested, the drug release percentages are 15 percent, 35 percent, 50 percent and 65 percent respectively. The experimental result shows that the drug-loaded coating of the stent manufactured in this embodiment has the controllable drug release property.

### Embodiment 8

Ethyl acetate is used as a solvent; polylactic acid with the weight average molecular weight of 200,000 and sirolimus are dissolved in the ethyl acetate at a mass ratio of 2:1 to form a solution; the surface of a pure iron stent substrate is coated with the solution, a drug-loaded coating with a thickness of 10 µm and an aperture less than 0.1 µm is formed; and the amount of the active drug on the area of the stent substrate is 150 µg/cm². The manufactured drug-loaded stents are implanted into the abdominal aorta of rabbits respectively, and samples are respectively taken on the 1st day, the 7th day, the 14th day and the 28th day and then are tested, the drug release percentages are 30 percent, 45 percent, 55 percent and 70 percent, respectively. The experimental result shows that the drug-loaded coating of the stent manufactured in this embodiment has the controllable drug release property.

### Contrast 1

Ethyl acetate is used as a solvent; polyglycolic acid with a weight average molecular weight of 30,000 and sirolimus are dissolved at a ratio of 1:1 to form a solution; the surface of a pure iron stent substrate is coated with the solution, thereby forming a fully-covering drug-loaded coating with a thickness of 40 µm and an aperture of 12 µm; and the amount of the active drug on the area of the stent substrate is 50 µg/cm². The manufactured drug-loaded stents are implanted into the abdominal aorta of rabbits, and samples are respectively taken at different time points; and then a drug release curve of the coating of the stent is tested, and the test result shows that the drug release percentage is more than 80 percent on the 1st day.

### Contrast 2

Trichloromethane is used as a solvent; polylactic acid with a weight average molecular weight of 20,000 and taxol are dissolved at a mass ratio of 10:1 to form a solution; the surface of a pure iron stent substrate is coated with the solution, thereby a drug-loaded coating with a thickness of 20 µm and an aperture less than 0.1 µm; and the amount of the active drug on the area of the stent substrate is 50 µg/cm². The manufactured drug-loaded stents are implanted into the abdominal aorta of a rabbit, and samples are respectively taken at different time points; and then a drug release curve of the coating of the stent is tested, and the test result shows that the drug release percentage is less than 3 percent on the 1st day.

It can be seen from the experimental results of the above embodiments 1 to 8, and the contrasts 1 to 2, that the drug-loaded coating of the iron-based alloy implantable medical device provided by the present application takes the polymer with the weight average molecular weight of more than or equal to 50,000 and less than or equal to 1,000,000 as a drug carrier. The drug-loaded coating having the micro-pores with the aperture not more than 10 microns is formed on the surface of the implantable medical device by adjusting the ratio of the polymer to the drug, the thickness of the drug-loaded coating, the drug amount and the type of the solvent, thereby realizing controllable drug release.

The above-mentioned embodiments only show several implementation modes of the present application, and their descriptions are more specific and detailed, but they are not be considered as limitations to the scope of the present application. It should be noted that an ordinary person skilled in the art may further make multiple transformations and improvements without departing from the scope of the present application, and these transformations and improvements shall all fall within the protection scope of the present application. Therefore, the protection scope of the present application shall be based on the attached claims.

## Claims

1. An iron-based alloy implantable medical device, comprising an iron-based alloy substrate and a drug-loaded coating, wherein the drug-loaded coating comprises a polymer and an active drug; the weight average molecular weight of the polymer is more than or equal to 50,000 and less than or equal to 1,000,000; and micro-pores having an aperture not more than 10 microns are formed in the drug-loaded coating.

2. The iron-based alloy implantable medical device according to claim 1, wherein the weight average molecular weight of the polymer is more than or equal to 100,000 and less than or equal to 500,000.

3. The iron-based alloy implantable medical device according to claim 1, wherein the thickness of the drug-loaded coating is more than or equal to 2 microns and less than or equal to 50 microns.

4. The iron-based alloy implantable medical device according to claim 1, wherein the amount of the active drug on a unit area of the iron-based alloy substrate is more than or equal to 5 µg/cm² and less than or equal to 500 µg/cm².

5. The iron-based alloy implantable medical device according to claim 1, wherein a mass ratio of the polymer to the active drug is more than or equal to 50:1 and less than or equal to 0.1:1.

6. The iron-based alloy implantable medical device according to claim 1, wherein a one-layer drug-loaded coating or a multiple-layer drug-loaded coating are provided.

7. The iron-based alloy implantable medical device according to claim 2, wherein the aperture of the micro-pores is not more than 1 micron, and further not more than 0.1 micron.

8. The iron-based alloy implantable medical device according to claim 7, wherein the thickness of the drug-loaded coating is more than or equal to 5 microns and less than or equal to 30 microns.

9. The iron-based alloy implantable medical device according to claim 8, wherein the amount of the active drug on the unit area of the iron-based alloy substrate is more than or equal to 50 µg/cm² and less than or equal to 300 µg/cm².

10. The iron-based alloy implantable medical device according to claim 9, wherein the mass ratio of the polymer to the active drug is more than or equal to 10:1 and less than or equal to 0.2:1.

11. The iron-based alloy implanted medical device according to claim 1, wherein the polymer is a degradable polyester polymer, or a blend of the degradable polyester polymer and a non-degradable polyester polymer, or a copolymer of at least one monomer forming the degradable polyester polymer and at least one monomer forming the non-degradable polyester polymer; the degradable polyester polymer is selected from the group consisting of polylactic acid, polyglycolic acid, poly(ethylene succinate), poly(P-hydroxybutyrate), polycaprolactone, polyethylene glycol adipate, poly-pentanoate, poly-hydroxyl alkyl alcohol ester and poly(malate), or a copolymer of at least two of monomers forming the degradable polyester polymer; and the non-degradable polyester polymer is selected from the group consisting of starch, chitosan, cellulose, glycan, glycan and a derivative thereof, polyurethane (PU), polycarbonate, polymethyl methacrylate (PMMA), polystyrene (PS), polybutylene, poly-butyl-methacrylate (PBMA) and polyacrylamide, or a copolymer of at least two of monomers forming the non-degradable polyester polymer.

12. The iron-based alloy implantable medical device according to claim 1, wherein the active drug is selected from the group consisting of a vascular proliferation inhibition drug, an antiplatelet drug, an antithrombotic drug, an anti-inflammation reaction drug and an antiallergic drug; the vascular proliferation inhibition drug is selected from the group consisting of taxol, sirolimus and a derivative thereof; the antiplatelet drug is cilostazol; the antithrombotic drug is heparin; the anti-inflammation reaction drug is dexamethasone; and the antiallergic drug is selected from the group consisting of diphenhydramine, chlorpheniramine, promethazine, dexamethasone, hydrocortisone, triamcinolone, methylprednisolone, clarityne, fexofenadine, levocetirizine, mizolastine and ebastine.

13. The iron-based alloy implantable medical device according to claim 1, wherein the iron-based alloy substrate is an iron-based alloy with the carbon content not more than 2.11 wt.% or pure iron.

14. The iron-based alloy implantable medical device according to claim 1, wherein the release percentage of the active drug is more than or equal to 4t^{1/2}-1 and less than or equal to 6.9t^{1/2}+63, and t is more than 0 and less than or equal to 28, and represents sampling time/days.

15. A manufacturing method of the iron-based alloy implantable medical device according to any one of the claims 1 to 14, comprising: dissolving a polymer and an active drug in an organic solvent to form a solution, and then coating an iron-based alloy substrate with the solution, wherein the organic solvent is selected from group consisting of trichloromethane, dichloromethane, ethyl acetate, tetrahydrofuran, acetone, methanol, ethanol, acetonitrile, 1,4-dioxane, dimethyl formamide and isopropanol.
